# EUROPEAN PATENT APPLICATION

(11) **EP 4 186 513 A1**
(43) Date of publication of application: **31.05.2023**
(21) Application number: 21211289.0
(22) Date of filing: 30.11.2021
(51) Int. Cl.: A61K 31/7032, A61K 31/702, A61K 35/20, A61K 38/40, A61K 31/575, A61K 31/716, A61P 25/00

(54) **USES OF COMPOSITIONS COMPRISING A PREBIOTIC COMPONENT**

(71) Applicant: MJN U.S. Holdings LLC, Evansville, IN 47721 (US)
(72) Inventor: CHICHLOWSKI, Maciej Witalis, Evansville, Indiana 47721 (US); COTTER, Jack, Slough, Berkshire, SL1 3UH (GB); FAWKES, Neil Malcom Andrew, Slough, Berkshire, SL1 3UH (GB); PANDEY, Neeraj, Slough, Berkshire, SL1 3UH (GB)
(74) Representative: Bovill, Sally Marina

(57) **Abstract**

A composition for use in reducing stress and/or enhancing mood in a subject is described. The composition comprises a prebiotic component comprising polydextrose (PDX) and galactooligosaccharides (GOS).

## Description

### Field of the Invention

The present application relates to a composition comprising a prebiotic component comprising polydextrose (PDX) and galactooligosaccharides (GOS). In particular, the present application relates to the use of the composition for reducing stress and/or enhancing mood in a subject.

### Background

In modern life, people are subjected to stress on a daily basis. Stress can cause physical conditions, such as headaches and digestive issues. It can also cause psychological and emotional strains, including confusion, anxiety, and depression.

Moreover, exposure to stress negatively affects sleep and the sleep-wake cycle. For example, experiencing work-related stressors, having low social support, or exposure to trauma can all disrupt sleep and the sleep/wake cycle.

Modulation of the gut microbiota is emerging as a potential strategy to support mental health, cognitive function and sleep.

The gut microbiota can be viewed as an actual body organ contributing to the well-being of the host organism. The composition of the gut microbiota is in constant flow under the influence of factors such as the diet, ingested drugs, the intestinal mucosa, the immune system, and the microbiota itself. It is believed that the gut microbiota communicates with the central nervous system through neural, endocrine and immune pathways in a process described as the microbiome-gut-brain axis. The microbiome-gut-brain-axis is a bidirectional communication network that links the enteric and central nervous systems.

The gut microbiome is related to the host's sleep and circadian rhythm. There is a bidirectional connection between the gut microbiome and sleep disorders, depression and anxiety, with stress being a common cause of those conditions. For example, stress exposure disrupts sleep (a process called circadian misalignment) and causes gut microbial imbalance (dysbiosis). On the other hand, nutritional interventions such as prebiotics are known to promote the growth of healthy gut microbiota. In turn, healthy gut microbiota promotes a healthy microbiome gut-brain axis and this may provide cognitive benefits in a subject.

There are reports of benefits in stress reduction through nutritional interventions with the prebiotics polydextrose (PDX) and galactooligosaccharides (GOS) in preclinical rat models (Thompson et al., Frontiers in Behavioural Neuroscience, 10: 240, 2017 and Mika et al., European Journal of Neuroscience, 1-16, 2016). The test diet in Thompson *et al.* (2017) also included bioactive milk fractions: milk fat globule membrane (MFGM) and lactoferrin and the test diet in Mika *et al.* (2016) also included lactoferrin. However, to the best of our knowledge, PDX and GOS have not yet been evaluated together in humans.

Compositions that reduce stress in a human subject would therefore be beneficial.

### Summary of Invention

According to a first aspect of the present invention there is provided a composition for use in reducing stress and/or enhancing mood in a subject, the composition comprising a prebiotic component comprising polydextrose (PDX) and galactooligosaccharides (GOS).

Preferably, the reduction in stress is an improvement in response to psychological stress.

Preferably, the reduction in stress is selected from a lower resting heart rate, a lower elevation in heart rate, a higher heart rate variability (HRV), a lower increase in blood pressure, a lower increase in salivary cortisol and/or a lower increase in serum cortisol.

Preferably, the reduction in stress is a lower level of perceived stress.

Preferably, the composition is administered once daily or twice daily.

Preferably, the subject is an adult.

Preferably, the prebiotic component consists of PDX and GOS.

Preferably a daily dosage of PDX is in the range of about 0.05 g/day to about 10.0 g/day and a daily dosage of GOS is in the range of about 0.05 g/day to about 10.0 g/day.

Preferably, the composition further comprises a fat or lipid source, wherein the fat or lipid source comprises polar lipids.

Preferably the polar lipids comprise at least one of sphingomyelin, gangliosides, phospholipids, cholesterol, 7-dehydrocholesterol, or any combination thereof.

Preferably, the fat or lipid source is a whey protein concentrate and/or the fat or lipid source comprises milk fat globule membrane (MFGM).

Preferably, a daily dosage of MFGM is in the range of about 0.05 g/day to about 20.0 g/day.

Preferably, the composition further comprises lactoferrin.

Preferably, a daily dosage of lactoferrin is in the range of about 1 mg/day to about 10.0 g/day.

Preferably, the composition is a nutritional composition.

Preferably, the composition is a liquid, a liquid concentrate, a powder, or a nutritional bar.

According to an aspect related to the first aspect of the present invention there is provided a method for reducing stress and/or enhancing mood in a subject, the method comprising administering a composition comprising an effective amount of a prebiotic component comprising polydextrose (PDX) and galactooligosaccharides (GOS).

According to an aspect related to the first aspect of the present invention there is provided the use of a composition comprising a prebiotic component comprising polydextrose (PDX) and galactooligosaccharides (GOS) in the manufacture of a medicament for reducing stress and/or enhancing mood in a subject.

### Brief Description of the Drawings

Embodiments of the invention will now be described, by way of example only, with reference to the following drawings in which:

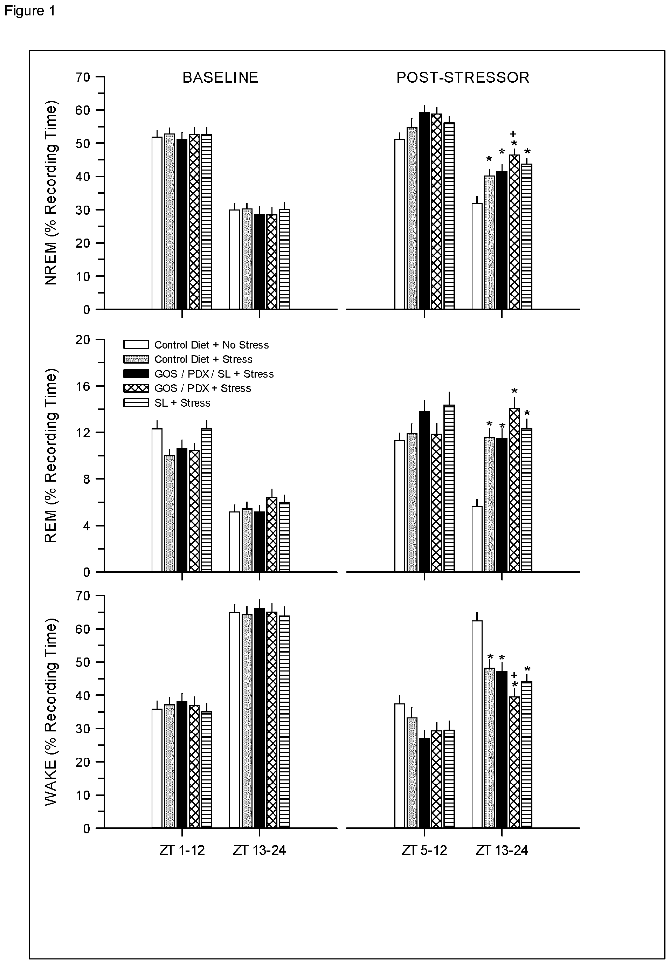

Figure 1 illustrates graphs showing the diurnal distribution of sleep-wake behaviour during undisturbed baseline (left panels) and for 18 hours following stressor (right panels) as detailed in Example 2.

### Definitions

*"Milk"* means a substance that has been drawn or extracted from the mammary gland of a mammal.

*"Milk-based composition"* means a composition comprising any mammalian milk-derived or mammalian milk-based product known in the art. For example, a "milk-based composition" may comprise bovine casein, bovine whey, bovine lactose, or any combination thereof.

*"Polar lipids",* in terms of the present disclosure, are the main constituents of natural membranes, occurring in all living organisms. The polar lipids in milk (i.e., milk polar lipids) are mainly situated in the milk fat globule membrane (MFGM). This is a highly complex biological membrane that surrounds the fat globule, hereby stabilising it in the continuous phase of the milk. Polar lipids are also present in other sources than milk such as eggs, meat and plants.

*"Enriched milk product"* generally refers to a milk ingredient that has been enriched with MFGM and/or certain MFGM components, such as proteins and lipids found in the MFGM.

*"Nutritional composition"* means a substance or composition that satisfies at least a portion of a human subject's nutrient requirements. *"Nutritional composition(s)"* may refer to liquids, powders, solutions, gels, pastes, solids, bars, concentrates, suspensions, ready-to-use forms of enteral formulas, oral formulas, formulas for infants, follow-up formulas, formulas for paediatric subjects, formulas for children, young child milks, and/or formulas for adults.

*"Reconstituted solution",* in terms of the present disclosure, means the solution prepared when a diluent (e.g. water, saline, etc.) is added to an ingredient (e.g. a powder, a solution, a gel, a suspension, a paste, a solid, a liquid, a liquid concentrate, etc.).

The term *"synthetic"* when applied to a composition, a nutritional composition, or a mixture means a composition, nutritional composition, or mixture obtained by biological and/or chemical means, which can be chemically identical to the mixture naturally occurring in mammalian milks. A composition, nutritional composition, or mixture is said to be *"synthetic"* if at least one of its components is obtained by biological (e.g. enzymatic) and/or chemical means.

*"Paediatric subject"* means a human subject under 18 years of age. The term *"adult",* in terms of the present disclosure, refers to a human subject that is 18 years of age or greater. The term *"paediatric subject"* may referto preterm infants, full-term infants, and/or children, as described below. A paediatric subject may be a human subject that is between birth and 8 years old. In another aspect, *"paediatric subject"* refers to a human subject between 1 and 6 years of age. Alternatively, *"paediatric subject"* refers to a human subject between 6 and 12 years of age.

*"Infant"* means a human subject ranging in age from birth to not more than one year and includes infants from 0 to 12 months corrected age. The phrase *"corrected age"* means an infant's chronological age minus the amount of time that the infant was born premature. Therefore, the corrected age is the age of the infant if it had been carried to full term. The term infant includes full-term infants, preterm infants, low birth weight infants, very low birth weight infants, and extremely low birth weight infants. *"Preterm"* means an infant born before the end of the 37^{th} week of gestation. *"Full-term"* means an infant born after the end of the 37^{th} week of gestation.

*"Child"* means a human subject ranging from 12 months to 13 years of age. A child may be a human subject between the ages of 1 and 12 years old. In another aspect, the terms *"children"* or *"child"* may refer to human subjects that are between 1 and about 6 years old. Alternatively, the terms *"children"* or *"child"* may referto human subjects that are between about 7 and about 12 years old. The term *"young child"* means a human subject ranging from 1 year to 3 years of age.

*"Infant formula"* means a composition that satisfies at least a portion of the nutrient requirements of an infant.

*"Follow-up formula"* means a composition that satisfies at least a portion of the nutrient requirements of an infant from the 6^{th} month onwards, and for young children from 1 to 3 years of age.

*"Young child milk",* in terms of the present disclosure, means a fortified milk-based beverage intended for children over one year of age (typically from one to six years of age). Young child milks are designed with the intent to serve as a complement to a diverse diet, to provide additional insurance that a child achieves continual, daily intake of all essential vitamins and minerals, macronutrients plus additional functional dietary components, such as non-essential nutrients that have purported health-promoting properties.

The term *"enteral"* means deliverable through or within the gastrointestinal, or digestive, tract. *"Enteral administration"* includes oral feeding, intragastric feeding, transpyloric administration, or any other administration into the digestive tract. *"Administration"* is broader than *"enteral administration"* and includes parenteral administration or any other route of administration by which a substance is taken into a subject's body.

*"Liquid concentrate",* in the context of the present disclosure, means a liquid that needs to be diluted before it is ready to be administered to, or consumed by, the subject.

The term *"prebiotic"* refers to a non-digestible food ingredient that beneficially affects the host by selectively stimulating the growth and/or activity of one or a limited number of bacteria in the digestive tract, which can improve the health of the host. Prebiotics exert health benefits, which may include, but are not limited to: selective stimulation of the growth and/or activity of one or a limited number of beneficial gut bacteria; stimulation of the growth and/or activity of ingested probiotic microorganisms; selective reduction in gut pathogens; and, favourable influence on gut short chain fatty acid profile. The prebiotic of the composition may be naturally occurring, synthetic, or developed through the genetic manipulation of organisms and/or plants, whether such new source is now known or developed later.

The *"gut"* of a subject may also be referred to as the gastrointestinal system, the gastrointestinal tract, the digestive system, and/or the digestive tract, of a subject.

The term *"human milk oligosaccharides"* or *"HMOs"* refers generally to a number of complex carbohydrates found in human breast milk.

*"Non-human lactoferrin"* refers to lactoferrin that is produced by or obtained from a source other than human breast milk.

The term *"sialic acid"* refers to a family of derivatives of neuraminic acid. N-acetylneuraminic acid (Neu5Ac) and N-glycolylneuraminic acid (Neu5Gc) are among the most abundant, naturally found forms of sialic acid, especially Neu5Ac in human and cow's milk.

The term *"degree of hydrolysis"* refers to the extent to which peptide bonds are broken by a hydrolysis method. The degree of protein hydrolysis for the purposes of characterising the hydrolysed protein component of the composition is easily determined by one of ordinary skill in the formulation arts, by quantifying the amino nitrogen to total nitrogen ratio (AN/TN) of the protein component of the selected composition. The amino nitrogen component is quantified by USP titration methods for determining amino nitrogen content, with the total nitrogen component being determined by the Kjeldahl method. These methods are well-known to one of ordinary skill in the analytical chemistry art.

The term *"partially hydrolysed"* means having a degree of hydrolysis which is greater than 0% but less than about 50%.

The term *"extensively hydrolysed"* means having a degree of hydrolysis which is greater than or equal to about 50%.

The term *"peptide"* describes linear molecular chains of amino acids, including single chain molecules or their fragments. The term "small amino acid peptide", in terms of this disclosure, means a peptide comprising no more than 50 total amino acids. The small amino acid peptides of the present disclosure may be naturally occurring, or they may be synthesised.

The term *"substantially free"* means containing less than a functional amount of the specified component, typically less than 0.1 % by weight, and includes 0% by weight of the specified ingredient.

*"Sleep duration",* in terms of the present disclosure, refers to the total amount of time defined as sleep, including non-rapid eye movement (NREM) sleep and rapid eye movement (REM) sleep. This may also be referred to as *"sleep quantity".*

*"Sleep quality",* in terms of the present disclosure, refers to the following measures of sleep as defined in the Pittsburgh Sleep Quality Index (Buysse et al., Psychiatry Research, 28(2), 193-213 (1989)): perceived sleep quality (also referred to as subjective sleep quality), sleep latency, sleep efficiency, sleep disturbance and daytime dysfunction.

The term *"epoch",* in the context of sleep, means a short interval of arbitrarily defined length (usually 20 to 60 seconds). The sleep stage or state of each consecutive epoch can be determined using polysomnography.

*"Sleep latency",* in terms of the present disclosure, means the time taken from intention to sleep to the first epoch of any stage of sleep. "Sleep latency" may also be referred to as *"sleep onset latency".*

*"REM latency",* in terms of the present disclosure, means the time from sleep onset to the first epoch of REM sleep.

*"Sleep efficiency",* in terms of the present disclosure, refers to the percentage of time in staged sleep from total sleep recorded time.

*"Sleep disturbance",* in terms of the present disclosure, refers to an awakening surrounded, at both ends, by any sleep stage.

*"Daytime dysfunction",* in terms of the present disclosure, is a measure of daytime sleepiness or difficulty in staying awake during the day.

All percentages, parts, and ratios as used herein are detailed by weight of the total composition, unless otherwise specified. All amounts specified as administered "per day" may be delivered in a single unit dose, in a single serving, or in two or more doses or servings administered over the course of a 24-hour period.

All references to singular characteristics or limitations in the present disclosure shall include the corresponding plural characteristic or limitation, and vice versa, unless otherwise specified or clearly implied to the contrary, by the context in which the reference is made.

All combinations of method or process steps disclosed herein can be performed in any order, unless otherwise specified or clearly implied to the contrary, by the context in which the referenced combination is made.

The compositions of the present disclosure can comprise, consist of, or consist essentially of any of the components described herein, as well as including any additional useful component.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings) may be replaced by alternative features serving the same, equivalent or similar purpose, unless expressly stated otherwise. Thus, unless expressly stated otherwise, each feature disclosed is one example only of a generic series of equivalent or similar features.

### Detailed Description

Prebiotics can beneficially affect host health by selectively stimulating the growth and/or activity of gut bacteria. Ingestion of prebiotics including polydextrose (PDX - a branch polymer of glucose and sorbitol) and galactooligosaccharides (GOS - a linear polymer of galactose) enhances the growth of lactic acid bacteria in the gastrointestinal tract.

PDX and GOS escape digestion by human enzymes and instead are fermented by the microbiota of the distal gut. While GOS is a short-chain prebiotic that is fermented rapidly, PDX is a more complex molecule that is fermented slowly and thus over a greater portion of the large intestine. It is therefore hypothesised that PDX and GOS work in a complementary and/or synergistic manner to alter the composition of the intestinal microbiota and confer a health benefit to the host.

Advantageously, the combination of PDX and GOS may also stimulate and/or enhance endogenous butyrate production by the gut microbiota, as well as other beneficial short-chain fatty acids (SCFAs) such as acetate and propionate. These metabolites act directly on the enteric nervous system and the vagus nerve (Petra et al., Clin Ther. 2015 May 1; 37(5): 984-995).

Preclinical data in Mika *et al.* (2016) suggest that early life diets containing PDX and GOS promote behavioural stress resistance. Rats were fed a test diet including PDX and GOS. PDX and GOS were found to alter the gut bacteria and increase *Lactobacillus* spp. in the gut. After four weeks, the rats were exposed to an acute inescapable stressor. The PDX and GOS test diet was found to reduce stress-evoked *cfos* mRNA in the dorsal raphe nucleus and modulate gene expression in neural circuits important in stress resistance.

Therefore, it is hypothesised that a prebiotic component comprising PDX and GOS would affect the central nervous system and improve a subject's response to stress by altering the gut microbiota and generating endogenous SCFAs including butyrate.

Further, Thompson *et al.* (2017) reports a preclinical study where rats were randomised to a test diet which differed from the control diet by the inclusion of PDX, GOS, milk fat globule membrane (MFGM) and lactoferrin. After exposure to an acute stressor, beneficial rapid-eye movement (REM) sleep rebounded more quickly and reductions in disruption of the circadian rhythm of core body temperature and microbiome dysbiosis were demonstrated in the test group of rats compared to the control group. Therefore, it is hypothesised that PDX and GOS may provide a stress-protective effect.

It is therefore hypothesised that a prebiotic component comprising PDX and GOS would work together in a complementary and/or synergistic manner to stimulate the growth and activity of beneficial gut microbiota. This optimises the composition of the gastrointestinal microbiota and supports the development of the microbiome-gut-brain axis. In this way, the composition comprising PDX and GOS is thought to lead to an improvement in emotional and cognitive indicators, particularly an improvement in stress response. This in turn would lead to an anti-anxiety effect and an enhancement in mood.

Therefore, the present invention provides a composition for use in reducing stress and/or enhancing mood in a subject, the composition comprising a prebiotic component comprising polydextrose (PDX) and galactooligosaccharides (GOS).

As outlined above, the prebiotic component comprising both PDX and GOS is hypothesised to reduce stress and/or enhance mood in a complementary and/or synergistic manner.

The reduction in stress may be a perceived (or subjective) improvement. For example, a subject may report a reduction in stress in their daily lives after consuming the composition. Alternatively, a reduction in stress may be a reduction in perceived stress, and this may be demonstrated using the Perceived Stress Scale (PSS) (Cohen et al., Journal of Health and Social Behavior, Vol. 24, No. 4 (Dec., 1983), 385-396).

Alternatively, the reduction in stress may be an improvement in a physiological measure of a subject's response to stress, such as psychological stress, compared to a subject receiving a control formulation. The improvement in a physiological measure of a subject's response to stress may be measured by at least one standardised clinical neuropsychological test. For example, the Trier Social Stress Test (TSST) (Kirschbaum et al., Neuropsychobiology 1993; 28, 76-81) may be used to measure the response to psychological stress. The TSST is a protocol for induction of moderate psychological stress in a laboratory setting and evaluates its effects on physiological responses such as heart rate, salivary cortisol levels and serum cortisol levels. Alternatively, the Observed Multitasking Stressor (OMS) may be used, as detailed in Example 1.

Alternatively, cortisol levels are known to peak after about 30 to 45 minutes after awakening in some subjects. Therefore, the cortisol awakening response (CAR) may be measured to provide a physiological biomarker of stress. Heart rate variability (HRV), which is a measure of the difference in time between each heartbeat, is also a physiological biomarker of stress, because people with a low HRV can easily experience acute stress while people with a high HRV rarely experience stress.

Therefore, the reduction in stress may be selected from a lower resting heart rate, a lower elevation in heart rate, a higher HRV, a lower increase in blood pressure, a lower increase in salivary cortisol and/or a lower increase in serum (e.g. blood) cortisol. Preferably, the reduction in stress is selected from a lower resting heart rate, a lower elevation in heart rate, a lower increase in blood pressure, a lower increase in salivary cortisol and/or a lower increase in serum (e.g. blood) cortisol.

The enhancement in mood may be a perceived (or subjective) enhancement in mood in the subject following consumption of the composition according to the present invention.

The subject is a human subject.

Preferably, the subject is at least three years of age, more preferably at least 4, more preferably at least 6, more preferably at least 12, more preferably at least 16 years of age. Most preferably, the subject is an adult.

The composition may be provided in a form suitable for enteral administration. The composition may be provided in the form of a tablet, a capsule, a powder, a solution, a suspension, a solid, a bar, a liquid, a liquid concentrate, or a reconstituted solution. Preferably, the composition is in the form of a liquid, a liquid concentrate, a powder or a nutritional bar. More preferably, the composition is in the form of a liquid, a powder or a nutritional bar.

The composition may be administered daily, for example once or twice daily. The composition may be in the form of a single daily dosage or portion. Alternatively, the total daily dosage of the composition may be administered in portions throughout the day e.g. two portions, three portions, etc. A "portion" may refer to a portion of about 15 g to about 50 g, or about 150 mL to about 500 mL, for example about 200 mL.

The composition may be consumed on consecutive days, for example for at least two three, four, 5, 6, 7, 14, 21, 28, 35, 42, 49 or at least 56 days.

The reduction in stress and/or enhancement in mood may be detectable after one daily dosage of the composition. Alternatively, the reduction in stress and/or enhancement in mood may only be detectable after, for example, daily dosages on at least two, three, four, 5, 6, 7, 14, 21, 28, 35, 42, 49 or at least 56 consecutive days.

In addition to providing benefits in relation to stress, preferably the administration of a composition according to the invention improves sleep duration, sleep quality and/or improves cognitive function in the subject.

The improvement in sleep quality may be selected from a reduction in sleep latency, an increase in sleep efficiency, a reduction in the number of sleep disturbances and/or an improvement in daytime dysfunction.

The improvement in cognitive function may an improvement in executive function, memory, attention, mental fatigue and/or mental processing speed. The improvement in memory may be an improvement in working memory, spatial memory and/or episodic memory.

In addition to the PDX and GOS, the prebiotic component may further comprise other oligosaccharides, polysaccharides, or any other prebiotics that comprise fructose, xylose, soya, galactose, glucose, mannose, or any combination thereof. More specifically, the prebiotic may further comprise lactulose, lactosucrose, raffinose, glucooligosaccharides, inulin, fructooligosaccharides, isomaltooligosaccharides, soybean oligosaccharides, lactosucrose, xylooligosaccharides, chitooligosaccharides, mannooligosaccharides, aribino-oligosaccharides, sialyloligosaccharides, fucooligosaccharides, and gentiooligosaccharides. The addition of one or more oligosaccharides, polysaccharides, or other prebiotics in addition to PDX and GOS enhances the growth of lactic acid bacteria in the gastrointestinal tract and further enhances the gut-brain axis.

The prebiotic component may comprise at least 20% weight per weight (w/w) PDX and GOS. Preferably, the prebiotic component consists of PDX and GOS.

The composition may comprise the prebiotic component in the range of about 1.0 g/L to about 40.0 grams per litre (g/L). Preferably, the composition comprises the prebiotic component in the range of about 2.0 g/L to about 36.0 g/L. Alternatively, the composition may comprise the prebiotic component in the range of about 0.01 g/100 kcal to about 16.0 g/100 kcal. Preferably, the composition comprises the prebiotic component in the range of about 0.1 g/100 kcal to about 10.0 g/100 kcal.

The composition may comprise PDX in the range of about 0.5 g/L to about 20.0 g/L of the composition. Preferably, the composition comprises PDX in the range of about 1.0 g/L to about 18.0 g/L. Alternatively, the composition may comprise PDX in the range of about 0.01 g/100 kcal to about 8.0 g/100 kcal. Preferably, the composition comprises PDX in the range of about 0.1 g/100 kcal to about 5.0 g/100 kcal.

PDX may be in the form of polydextrose powder.

The composition may comprise GOS in the range of about 0.5 g/L to about 20.0 g/L of the composition. Preferably, the composition comprises GOS in the range of about 1.0 g/L to about 18.0 g/L. Alternatively, the composition may comprise GOS in the range of about 0.01 g/100 kcal to about 8.0 g/100 kcal. Preferably, the composition comprises GOS in the range of about 0.1 g/100 kcal to about 5.0 g/100 kcal.

The ratio of PDX:GOS in the composition may be in the range of about 9:1 to about 1:9. Preferably, the ratio of PDX:GOS in the composition is in the range of about 5:1 to about 1:5. More preferably, the ratio of PDX:GOS in the composition is in the range of about 1:3 to about 3:1. Even more preferably, the ratio of PDX:GOS in the composition is about 1:1. Alternatively, the ratio of PDX:GOS in the composition is about 4:1.

PDX and GOS may be included in the composition in a total amount in the range of about 0.1 g/L to about 40.0 g/L. Preferably, the composition comprises PDX and GOS in a total amount in the range of about 2.0 g/L to about 36.0 g/L.

Alternatively, the composition may comprise PDX and GOS in a total amount of at least about 0.015 g/100 kcal. The composition may comprise PDX and GOS in a total amount in the range of about 0.01 g/100 kcal to about 16.0 g/100 kcal. Preferably, the composition comprises PDX and GOS in a total amount in the range of about 0.1 g/100 kcal to about 10.0 g/100 kcal.

A daily dosage of PDX may be in the range of about 0.05 g/day to about 10.0 g/day and a daily dosage of GOS may be in the range of about 0.05 g/day to about 10.0 g/day. Preferably, the daily dosage of PDX is in the range of about 1.0 g/day to about 6.0 g/day and the daily dosage of GOS is in the range of about 1.0 g/day to about 6.0 g/day. More preferably, the daily dosage of PDX is in the range of about 2 g/day to about 5 g/day and the daily dosage of GOS is in the range of about 2 g/day to about 5 g/day. The daily dosage of PDX and GOS may be varied depending on, for example, the requirement of the subject and the severity of the lack of sleep experienced by the subject. The dose of PDX and GOS may be in the form of a single daily dosage. Alternatively, the total daily dosage may be administered in portions throughout the day e.g. two portions, three portions, etc.

Preferably, the composition further comprises a fat or lipid source, wherein the fat or lipid source comprises polar lipids.

Polar lipids, especially those found in milk, are composed of three major groups of lipids:
(i) glycerophospholipids such as phosphatidylcholine (PC), phosphatidylethanolamine (PE), phosphatidylserine (PS), and phosphatidylinositol (PI), and their derivatives, (ii) sphingoids or sphingolipids such as sphingomyelin (SM) and glycosphingolipids comprise of cerebrosides (neutral glycosphingolipids containing uncharged sugars) and the gangliosides (GG, acidic glycosphingolipids containing sialic acid) and their derivatives, and (iii) cholesterol and its derivatives. The polar lipids in milk (i.e., milk polar lipids) are mainly situated in the milk fat globule membrane (MFGM).

MFGM is a naturally occurring bioactive membrane structure that surrounds the fat droplets in human milk and other mammalian milk e.g. cow's milk. MFGM is comprised of a trilayer lipid structure that comprises a complex mixture of phospholipids (such as phosphatidylethanolamine, phosphatidylcholine, phosphatidylserine, and phosphatidylinositol), glycolipids, glycosphingolipids (such as sphingomyelin and gangliosides), other polar lipids, proteins, glycoproteins (such as xanthine dehydrogenase, xanthine oxidase, lactadherin, fatty acid binding proteins (FABPs), mucin-1 (MUC-1), butyrophilins, adipophilin, and cluster of differentiation 36 (CD36)), triglycerides, cholesterol, 7-dehydrocholesterol, enzymes, and other components.

A significant body of preclinical data have demonstrated that MFGM, or components thereof, play roles in neural development and function, gastrointestinal immune defence and gut health. Glycosylated proteins (mucin-1, mucin-15, butyrophilin, and lactadherin) and glycosylated sphingolipids from MFGM may promote the development of healthy infant gut microbiota by favouring beneficial *Bifidobacterium* species (Bourlieu et al., Current Opinion in Clinical Nutrition and Metabolic Care: March 2015, 18(2), 118-127).

Clinical reports also suggest that MFGM and components thereof may affect brain function directly (Thompson *et al.* 2017; Schubert et al. Nutr Res. 2011; 31: 413-420). For example, Schubert *et al.* suggests that phospholipids may have cognitive benefits for those that have high and/or chronic stress.

Accordingly, the inclusion of a fat or lipid source, wherein the fat or lipid source comprises polar lipids, is thought to help promote the growth of beneficial gut microbial species and in turn promotes a healthy microbiome gut-brain-axis. It is therefore hypothesised that the fat or lipid source would work with the prebiotic component comprising PDX and GOS in a complementary and/or synergistic manner.

The fat and/or lipid source may comprise animal sources, such as milk fat, butter, butter fat, egg yolk lipid; marine sources, such as fish oils, marine oils, single cell oils; vegetable and plant oils, such as corn oil, canola oil, sunflower oil, soybean oil, palm olein oil, coconut oil, high oleic sunflower oil, evening primrose oil, rapeseed oil, olive oil, flaxseed (linseed) oil, cottonseed oil, high oleic safflower oil, palm stearin, palm kernel oil, wheat germ oil; medium chain triglyceride oils and emulsions and esters of fatty acids; and any combinations thereof. The fat or lipid source may be present in the composition in addition to another fat or lipid source, such as a source of long chain polyunsaturated fatty acids (LCPUFA).

The polar lipids may comprise phospholipids (such as phosphatidylethanolamine, phosphatidylcholine, phosphatidylserine, and phosphatidylinositol), glycolipids, glycosphingolipids (such as sphingomyelin and gangliosides), cholesterol, 7-dehydrocholesterol, or any combination thereof.

The amount of the fat or lipid source in the composition may be sufficient so as to provide at least 0.5 mg of gangliosides. The amount of the fat or lipid source in the composition may be sufficient so as to provide 0.5 mg to 3 g of gangliosides; preferably, 6 mg to 600 mg of gangliosides. The amount of the fat or lipid source in the composition may be sufficient so as to provide at least 1.5 mg of cholesterol. The amount of the fat or lipid source in the composition may be sufficient so as to provide 1.5 mg to 9 g of cholesterol; preferably, 50 mg to 5 g of cholesterol. The amount of the fat or lipid source in the composition may be sufficient so as to provide at least 5 µg of 7-dehydrocholesterol. The amount of the fat or lipid source in the composition may be sufficient so as to provide 5 µg to 100 mg of 7-dehydrocholesterol; preferably, 20 µg to 50 mg of 7-dehydrocholesterol.

The amount of fat or lipid source in the composition may be sufficient so as to provide at least 10 mg of sphingomyelin. The amount of the fat or lipid source in the composition may be sufficient so as to provide at least 50 mg of phospholipids. The amount of the fat or lipid source in the composition may be sufficient so as to provide 10 mg to 5 g of sphingomyelin; preferably, 50 mg to 1 g of sphingomyelin. The amount of the fat or lipid source in the composition may be sufficient so as to provide 50 mg to 30 g of phospholipids; preferably, 250 mg to 10 g of phospholipids.

In a preferred embodiment, the fat or lipid source is a whey protein concentrate (WPC). The whey protein concentrate may be provided in any suitable form for making a synthetic nutritional composition, preferably the whey protein concentrate is provided in powdered form or provided in a liquid form.

The composition may comprise WPC at a level of about 0.5 g/L to about 40 g/L. Preferably, the WPC is present at a level of about 1 g/L to about 35 g/L. Alternatively, the composition may comprise WPC at a level of about 0.06 g/100 kcal to about 20 g/100 kcal. Preferably, the WPC is present at a level of about 0.3 g/100 kcal to about 15 g/100 kcal. More preferably, the WPC is present in the composition at a level of about 0.4 g/100 kcal to about 10 g/100 kcal.

The amount of the WPC in the composition may be sufficient so as to provide at least 0.5 mg of gangliosides. The amount of the WPC in the composition may be sufficient so as to provide 0.5 mg to 3 g of gangliosides; preferably, 6 mg to 600 mg of gangliosides. The amount of the WPC in the composition may be sufficient so as to provide at least 1.5 mg of cholesterol. The amount of the WPC in the composition may be sufficient so as to provide 1.5 mg to 9 g of cholesterol; preferably, 50 mg to 5 g of cholesterol. The amount of the WPC in the composition may be sufficient so as to provide at least 5 µg of 7-dehydrocholesterol. The amount of the WPC in the composition may be sufficient so as to provide 5 µg to 100 mg of 7-dehydrocholesterol; preferably, 20 µg to 50 mg of 7-dehydrocholesterol.

The amount of WPC in the composition may be sufficient so as to provide at least 10 mg of sphingomyelin. The amount of the WPC in the composition may be sufficient so as to provide at least 50 mg of phospholipids. The amount of the WPC in the composition may be sufficient so as to provide at least 50 mg of glycoproteins. The glycoproteins may comprise xanthine dehydrogenase, xanthine oxidase, lactadherin, fatty acid binding proteins (FABPs), mucin-1, butyrophilins, adipophilin, and cluster of differentiation (CD36), or any combination thereof. The amount of the WPC in the composition may be sufficient so as to provide 10 mg to 5 g of sphingomyelin; preferably, 50 mg to 1 g of sphingomyelin. The amount of the WPC in the composition may be sufficient so as to provide 50 mg to 30 g of phospholipids; preferably, 250 mg to 10 g of phospholipids. The amount of the WPC in the composition may be sufficient so as to provide 50 mg to 30 g of glycoproteins; preferably, 250 mg to 10 g of glycoproteins.

In a preferred embodiment, the fat or lipid source comprises MFGM.

MFGM may be present in the composition in an amount of at least about 10 mg/100 kcal. The composition may comprise MFGM in the range of about 10 mg/100 kcal to about 20 g/100 kcal. Preferably, the composition comprises MFGM in the range of about 15 mg/100 kcal to about 15 g/100 kcal. More preferably, the composition comprises MFGM in the range of about 20 mg/100 kcal to about 10 g/100 kcal.

Alternatively, the composition may comprise MFGM in the range of about 0.1 g/L to about 40 g/L. Preferably, the composition comprises MFGM in the range of about 0.6 g/L to about 35 g/L.

The MFGM may be provided by a whey protein concentrate. The MFGM may be provided by an enriched milk product, buttermilk, or a combination thereof. The enriched milk product may be formed by fractionation of non-human milk, such as bovine milk. The enriched milk product may have a total protein level in a range of between 20% and 90%; preferably, the enriched milk product has a total protein level in a range of between 50% and 80%.

The enriched milk product may comprise an enriched whey protein concentrate (eWPC). Alternatively, the enriched milk product may comprise an enriched lipid fraction derived from milk. The eWPC and the enriched lipid fraction may be produced by any number of fractionation techniques. These techniques comprise, but are not limited, to melting point fractionation, organic solvent fractionation, super critical fluid fractionation, and any variants and/or any combination thereof. Alternatively, eWPC is available commercially, including under the trade name Lacprodan MFGM-10, available from Aria Food Ingredients.

The MFGM may be provided by buttermilk. Buttermilk, in the context of the present disclosure, refers to an aqueous by-product of different milk fat manufacturing processes, especially the butter making process. Buttermilk is a concentrated source of MFGM components compared to other milk sources. Buttermilk includes dry buttermilk, which is defined as having a protein content of not less than 30%, and dry buttermilk product, which is defined as having a protein content of less than 30%. Both types of dry buttermilk have a minimum fat content of 4.5% and a moisture maximum of 5%. Cultured buttermilk is also within the contemplation of this disclosure. Buttermilk contains components such as lactose, minerals, oligosaccharides, immunoglobulins, milk lipids, and milk proteins, each of which is found in the aqueous phase during certain dairy cream processing steps.

Buttermilk may be obtained through different processes, such as: churning of cream during production of butter or cheese; production of variants of butter such as sweet cream butter, clarified butter, butterfat; production of anhydrous milk fat (butter oil) from cream or butter; removal of the fat-free dry matter and water from milk, cream, or butter, which is required to make anhydrous milk fat, yields buttermilk as a by-product; removal can be accomplished by mechanical- and/or chemical-induced separation; or, production of anhydrous milk fat (butter oil) from blending secondary skim and β-serum (and/or butter serum) streams together, respectively.

The enriched milk product, buttermilk, or both may be derived from non-human milk sources, such as bovine whole milk, bovine cream, porcine milk, equine milk, buffalo milk, goat milk, murine milk, camel milk, or any combination thereof.

The composition may comprise eWPC at a level of about 0.5 g/L to about 40 g/L. Preferably, the eWPC is present at a level of about 1 g/L to about 35 g/L. Alternatively, the composition may comprise eWPC at a level of about 0.06 g/100 kcal to about 20 g/100 kcal. Preferably, the eWPC is present at a level of about 0.3 g/100 kcal to about 15 g/100 kcal. More preferably, the eWPC is present in the composition at a level of about 0.4 g/100 kcal to about 10 g/100 kcal.

The composition may comprise buttermilk at a level of about 0.5 grams per litre (g/L) to about 70 g/L. Preferably, the buttermilk is present at a level of about 1 g/L to about 60 g/L. More preferably, buttermilk is present in the composition at a level of about 3 g/L to about 50 g/L. Alternatively, the composition may comprise buttermilk at a level of about 0.06 grams per 100 kilocalories (g/100 kcal) to about 20 g/100 kcal. Preferably, the buttermilk is present at a level of about 0.3 g/100 kcal to about 15 g/100 kcal. More preferably, the buttermilk is present in the composition at a level of about 0.4 g/100 kcal to about 10 g/100 kcal.

The amount of MFGM in the composition may be sufficient so as to provide at least 0.5 mg of gangliosides. The amount of MFGM in the composition may be sufficient so as to provide 0.5 mg to 3 g of gangliosides; preferably, 6 mg to 600 mg of gangliosides. The amount of MFGM in the composition may be sufficient so as to provide at least 1.5 mg of cholesterol. The amount of MFGM in the composition may be sufficient so as to provide 1.5 mg to 9 g of cholesterol; preferably, 50 mg to 5 g of cholesterol. The amount of MFGM in the composition may be sufficient so as to provide at least 5 µg of 7-dehydrocholesterol. The amount of MFGM in the composition may be sufficient so as to provide 5 µg to 100 mg of 7-dehydrocholesterol; preferably, 20 µg to 50 mg of 7-dehydrocholesterol.

The amount of MFGM in the composition may be sufficient so as to provide at least 10 mg of sphingomyelin. The amount of MFGM in the composition may be sufficient so as to provide at least 50 mg of phospholipids. The amount of MFGM in the composition may be sufficient so as to provide at least 50 mg of glycoproteins. The glycoproteins may comprise xanthine dehydrogenase, xanthine oxidase, lactadherin, fatty acid binding proteins (FABPs), mucin-1, butyrophilins, adipophilin, and cluster of differentiation (CD36), or any combination thereof. The amount of MFGM in the composition may be sufficient so as to provide 10 mg to 5 g of sphingomyelin; preferably, 50 mg to 1 g of sphingomyelin. The amount of MFGM in the composition may be sufficient so as to provide 50 mg to 30 g of phospholipids; preferably, 250 mg to 10 g of phospholipids. The amount of MFGM in the composition may be sufficient so as to provide 50 mg to 30 g of glycoproteins; preferably, 250 mg to 10 g of glycoproteins.

A daily dosage of MFGM may be in the range of about 0.05 g/day to about 20.0 g/day, preferably about 5 g/day to about 15 g/day, more preferably about 10 g/day. The daily dosage may be varied depending on, for example, the requirement of the subject and the severity of the lack of sleep experienced by the subject. The dose of MFGM may be in the form of a single daily dosage. Alternatively, the total daily dosage may be administered in portions throughout the day e.g. two portions, three portions, etc.

Preferably, the composition further comprises lactoferrin.

Lactoferrin is a multifunctional iron-binding glycoprotein naturally present in milk. The lactoferrin may comprise human lactoferrin produced by a genetically modified organism, humanised lactoferrin, non-human lactoferrin, or a combination thereof. The non-human lactoferrin may comprise bovine lactoferrin, porcine lactoferrin, equine lactoferrin, buffalo lactoferrin, goat lactoferrin, murine lactoferrin, or camel lactoferrin.

Lactoferrin is known to have antimicrobial properties. Preclinical data reported in Mastromarino et al., Biometals 27, 1077-1086 (2014) suggest a potential modulatory role for lactoferrin on the gut microbiota.

There is also emerging preclinical evidence for a potential role of lactoferrin in brain development and cognitive functions. Clinical reports suggest that lactoferrin may affect brain function directly (Thompson *et al.* 2017). Further, preclinical data in Mika *et al.* (2016), suggest that early life diets containing lactoferrin promote behavioural stress resistance. Rats were fed a test diet including lactoferrin. Lactoferrin was found to alter the gut bacteria. After four weeks, the rats were exposed to an acute inescapable stressor. The lactoferrin test diet was found to reduce stress-evoked *cfos* mRNA in the dorsal raphe nucleus and modulate gene expression in neural circuits important in stress resistance.

The inclusion of lactoferrin is thought to optimise the composition of the gut microbiota and improve the stress response in a subject. It is therefore hypothesised that lactoferrin would work with the prebiotic component comprising PDX and GOS in a complementary and/or synergistic manner.

The composition may comprise lactoferrin in the range of about 0.01 grams per litre (g/L) to about 3.0 grams per litre (g/L). Preferably, the composition comprises lactoferrin in the range of about 0.1 g/L to about 2.0 g/L.

Alternatively, the lactoferrin may be administered as a reconstituted solution comprising lactoferrin in the range of about 15 milligrams per 100 kilocalories (mg/100 kcal) to about 10.0 grams per 100 kilocalories (g/100 kcal). Preferably, the reconstituted solution comprises lactoferrin in the range of about 0.1 g/100 kcal to about 1.0 g/100 kcal.

The daily dosage of lactoferrin may be in the range of about 1 mg/day to about 10.0 g/day. More preferably, the daily dosage of lactoferrin is in the range of about 1 mg/day to about 5.0 g/day. More preferably, the daily dosage of lactoferrin is in the range of about 5 mg/day to about 2.0 g/day. Even more preferably, the daily dosage of lactoferrin is in the range of about 0.4 g/day to about 0.8 g/day, most preferably about 0.6 g/day. The dose of lactoferrin may be in the form of a single daily dosage. Alternatively, the total daily dosage may be administered in portions throughout the day e.g. two portions, three portions, etc.

In a particularly preferred composition, the composition comprises PDX, GOS and at least one of fat or lipid source comprising polar lipids and lactoferrin. In a more particularly preferred composition, the composition comprises PDX, GOS, the fat or lipid source and lactoferrin. The particularly preferred composition may comprise any aspect of the fat or lipid source and lactoferrin detailed above. For the reasons described previously, it is believed that a composition comprising the combination of PDX, GOS, the fat or lipid source and lactoferrin will result in an even greater complementary and/or synergistic effect in the reduction of stress and/or enhancement of mood in a subject.

In a particularly preferred embodiment, the daily dosage of PDX is in the range of about 2 g/day to about 5 g/day, the daily dosage of GOS is in the range of about 2 g/day to about 5 g/day, the daily dosage of MFGM is about 5 g/day to about 15 g/day and the daily dosage of lactoferrin is in the range of about 0.4 g/day to about 0.8 g/day.

The composition may comprise a protein source, a carbohydrate source, or any combination thereof. The composition may comprise one or more: source of long chain polyunsaturated fatty acids (LCPUFAs); human milk oligosaccharides (HMOs) in addition to GOS; β-glucan; sialic acid; suitable composition ingredient; or, any combination thereof.

The composition may comprise at least one protein source, in addition to lactoferrin, a whey protein concentrate or MFGM, wherein the protein source provides further protein to the composition. The protein source may comprise intact protein, partially hydrolysed protein, extensively hydrolysed protein, small amino acid peptides, or any combination thereof. The protein source may be derived from any mammalian animal milk protein or plant protein, as well as their fractions, or any combination thereof. The protein source may comprise bovine milk, caprine milk, whey protein, casein protein, soy protein, rice protein, pea protein, peanut protein, egg protein, sesame protein, fish protein, wheat protein, hydrolysed protein, or any combination thereof. Bovine milk protein sources may comprise, but are not limited to, milk protein powders, milk protein concentrates, milk protein isolates, non-fat milk solids, non-fat milk, non-fat dry milk, whey protein, whey protein isolates, sweet whey, acid whey, casein, acid casein, caseinate (e.g. sodium caseinate, sodium calcium caseinate, calcium caseinate), or any combination thereof.

The composition may comprise at least one further carbohydrate source, in addition to PDX and GOS. The carbohydrate source may comprise lactose, glucose, fructose, maltodextrins, sucrose, starch, maltodextrin, maltose, fructooligosaccharides, corn syrup, high fructose corn syrup, dextrose, corn syrup solids, rice syrup solids, sucralose, glycerol, maltitol, maltitol syrup, xylitol, honey, oligofructose syrup or any combination thereof. Moreover, hydrolysed, partially hydrolysed, and/or extensively hydrolysed carbohydrates may be desirable for inclusion in the composition due to their easy digestibility. More specifically, hydrolysed carbohydrates are less likely to contain allergenic epitopes. The composition may therefore comprise a carbohydrate source comprising hydrolysed or intact, naturally or chemically modified, starches sourced from corn, tapioca, rice, or potato, in waxy or non-waxy forms, such as hydrolysed corn starch.

The composition may further comprise one or more human milk oligosaccharides (HMOs) selected from the group comprising 2'-fucosyllactose (2FL), 3'-fucosyllactose (3FL), lacto-N-tetraose (LNT), lacto-N-neotetraose (LNnT), lacto-N-fucopentaose I (LNFP-I), 3'-sialyllactose (3SL), 6'-sialyllactose (6SL), or a combination thereof.

The composition may comprise an HMO in addition to PDX and GOS in the range of about 0.01 g/L to about 5.0 g/L. Preferably, the composition comprises an HMO in the range of about 0.05 g/L to about 4.0 g/L of the composition. More preferably, the composition comprises an HMO in the range of about 0.05 g/L to about 2.0 g/L of the composition. Alternatively, the composition may comprise an HMO in the range of about 0.01 g/100 kcal to about 2.0 g/100 kcal. Preferably, the composition comprises an HMO in the range of about 0.01 g/100 kcal to about 1.5 g/100 kcal.

The composition may comprise a source of long-chain polyunsaturated fatty acids (LCPUFAs). The source of LCPUFAs may comprise docosahexaenoic acid (DHA), α-linoleic acid, γ-linoleic acid, linoleic acid, linolenic acid, eicosapentaenoic acid (EPA), arachidonic acid (ARA), or any combination thereof. Preferably, the composition comprises a source of LCPUFAs comprising DHA, ARA, or a combination thereof.

The composition may comprise an LCPUFA in an amount of at least about 5 mg/100 kcal. The composition may comprise an LCPUFA in the range of about 5 mg/100 kcal to about 100 mg/100 kcal. Preferably, the composition comprises an LCPUFA in the range of about 10 mg/100 kcal to about 50 mg/100 kcal.

The composition may comprise DHA in the range of about 5 mg/100 kcal to about 80 mg/100 kcal. Preferably, the composition comprises DHA in the range of about 10 mg/100 kcal to about 20 mg/100 kcal. More preferably, the composition comprises DHA in the range of about 15 mg/100 kcal to about 20 mg/100 kcal.

The composition may comprise ARA in the range of about 10 mg/100 kcal to about 100 mg/100 kcal of ARA. Preferably, the composition comprises ARA in the range of about 15 mg/100 kcal to about 70 mg/100 kcal. More preferably, the composition comprises ARA in the range of about 20 mg/100 kcal to about 40 mg/100 kcal.

The composition may comprise both DHA and ARA. The weight ratio ofARA:DHA may be in the range of about 1:3 to about 9:1. Preferably, the weight ratio of ARA:DHA is in the range of about 1:2 to about 4:1. The composition may comprise oils containing DHA and/or ARA. If utilised, the source of DHA and/or ARA may be any source known in the art such as marine oil, fish oil, single cell oil, egg yolk lipid, or brain lipid. The DHA and ARA may be sourced from single cell oils, DHASCO^{®} and ARASCO^{®} from DSM Nutritional Products, or variations thereof. The DHA and ARA may be in a natural form, provided that the remainder of the LCPUFA source does not result in any substantial deleterious effect on the subject. Alternatively, the DHA and ARA may be used in refined form.

The composition may comprise β-glucan. Preferably, the β-glucan comprises β-1,3-glucan. Preferably, the β-1,3-glucan comprises β-1,3;1,6-glucan. The composition may comprise β-glucan present in the range of about 0.010 grams to about 0.080 grams per 100g of composition. Alternatively, the composition may comprise β-glucan in the range of about 3 mg/100 kcal to about 17 mg/100 kcal. Preferably, the composition comprises β-glucan in the range of about 4 mg/100 kcal to about 17 mg/100 kcal.

The composition may comprise sialic acid. Mammalian brain tissue contains the highest levels of sialic acid as sialic acid is incorporated into brain-specific proteins, such as the neural cell adhesion molecule (NCAM) and lipids (e.g. gangliosides). Sialic acid is therefore believed to play an important role in neural development and function, learning, cognition, and memory.

The composition may comprise sialic acid provided by an inherent source (such as eWPC), exogenous sialic acid, sialic acid from sources (such as cGMP), or any combination thereof. The composition may comprise sialic acid in the range of about 100 mg/L to about 800 mg/L. Preferably, the composition comprises sialic acid in the range of about 120 mg/L to about 600 mg/L. More preferably, the composition comprises sialic acid in the range of about 140 mg/L to about 500 mg/L. Alternatively, the composition may comprise sialic acid in the range of about 1 mg/100 kcal to about 120 mg/100 kcal. Preferably, the composition comprises sialic acid in the range of about 14 mg/100 kcal to about 90 mg/100 kcal. More preferably, the composition comprises sialic acid in the range of about 15 mg/100 kcal to about 75 mg/100 kcal.

The composition may comprise one or more suitable composition ingredient, wherein the suitable composition ingredient comprises a vitamin including folic acid, a mineral, arginine, choline, inositol, an emulsifier, a preservative, a stabiliser, a colouring agent, a flavouring agent, a flow agent, stevia, almond flour, citric acid, or a combination thereof. The composition may comprise choline. Choline is a nutrient that is essential for normal function of cells. Choline is a precursor for membrane phospholipids and it accelerates the synthesis and release of acetylcholine, a neurotransmitter involved in memory storage. Without wishing to be bound by theory, it is believed that dietary choline and docosahexaenoic acid (DHA) act synergistically to promote the biosynthesis of phosphatidylcholine and thus, help promote synaptogenesis in human subjects. Additionally, choline and DHA act synergistically to promote dendritic spine formation, which is important in the maintenance of established synaptic connections. The composition may comprise about 20 mg to about 100 mg of choline per 8 fl. oz. (236.6 mL) serving.

The composition may comprise inositol. The inositol may be present as exogenous inositol, inherent inositol, or a combination thereof. The composition may comprise inositol in the range of about 10 mg/100 kcal to 40 mg/100 kcal. Preferably, the composition comprises inositol in the range of about 20 mg/100 kcal to 40 mg/100 kcal. Alternatively, the composition comprises inositol in the range of about 130 mg/L to about 300 mg/L.

The composition may comprise one or more emulsifiers, as an emulsifier can increase the stability of the composition. The emulsifier may comprise, but is not limited to, egg lecithin, soy lecithin, alpha lactalbumin, monoglycerides, diglycerides, or any combination thereof.

The composition may comprise one or more preservatives, as a preservative can extend the shelf-life of the composition. The preservative may comprise, but is not limited to, potassium sorbate, sodium sorbate, potassium benzoate, sodium benzoate, calcium disodium EDTA, or any combination thereof.

The composition may comprise one or more stabilisers, as a stabiliser can help preserve the structure of the composition. The stabiliser may comprise, but is not limited to, gum arabic, gum ghatti, gum karaya, gum tragacanth, agar, furcellaran, guar gum, gellan gum, locust bean gum, pectin, low methoxyl pectin, gelatine, microcrystalline cellulose, CMC (sodium carboxymethylcellulose), methylcellulose hydroxypropyl methyl cellulose, hydroxypropyl cellulose, DATEM (diacetyl tartaric acid esters of mono- and diglycerides), dextran, carrageenans, or any combination thereof.

The composition may be intended for an adult or a child. Preferably the composition is intended for an adult.

The composition may be a nutritional composition. The composition may comprise a nutritional supplement, an adult's nutritional product, a children's nutritional product, a young child milk, or any other composition designed for a paediatric subject. The composition may be provided in an orally-ingestible form, wherein *"orally-ingestible"* comprises a food, a nutritional bar, a beverage, a tablet, a capsule, or a powder. The composition may be provided as a ready-to-use product or it may be provided in a powder form or a concentrated form, such as a liquid concentrate. For example, the composition may be provided in powder form with a particle size in the range of 5 µm to 1500 µm, more preferably in the range of 10 µm to 1000 µm, and even more preferably in the range of 50 µm to 300 µm.

Preferably, the composition is a synthetic composition.

The composition may be suitable for a number of dietary requirements. The composition may be kosher. The composition may be a non-genetically modified product. The composition may be sucrose-free. The composition may also be lactose-free. The composition may not contain any medium-chain triglyceride oil. No carrageenan may be present in the composition. The composition may be free of all gums.

The invention is not restricted to the details of the foregoing embodiment(s). The invention extends to any novel one, or any novel combination, of the features disclosed in this specification (including any accompanying claims, abstract and drawings), or to any novel one, or any novel combination, of the steps of any method or process so disclosed.

The scope of the present invention is defined in the appended claims. It is to be understood that the skilled person may make amendments to the scope of the claims without departing from the scope of the present disclosure.

### Experimental Section

### Example 1 - Clinical Study

This example describes a randomised, double-blind, placebo-controlled, four-arm parallel group clinical study that will be carried out to assess the sleep, gut microbiome, cognitive, immune and stress effects of 56 days administration of three formulations of three prebiotic-based investigational supplements, in comparison to a placebo control, in a cohort of healthy adults reporting poor sleep quality. In addition, some of these effects will also be measured during the administration period to monitor the time course of any observed changes, as detailed below.

### Study Design

The main criteria for inclusion will be healthy adults aged 25 to 60 years reporting as 'poor sleepers'. 'Poor sleep' is defined based on both a subjective perception (e.g., poor subjective sleep quality, unrefreshing sleep) and a total score of >5 on the Pittsburgh Sleep Quality Index (PSQI).

The exclusion criteria will include current or recent sleep disorders, including sleep apnoea and insomnia. This will be assessed by means of the Sleep Disorders Symptom Checklist-25 (SDS-CL-25; Klingman et al., Sleep Med Res. 2017;8(1):17-25). If a participant reports positively to any of the 25 questions in terms of being affected for three nights per week, or more, this will be followed up using a clinical interview according to the International Classification of Sleep Disorders (ICSD-3) to exclude on the basis of a sleep disorder.

Participants who are poor sleepers will be randomised (N=17 per arm, stratified by age: 25-39 years, N=9 and 40-60 years, N=8) to receive one of three test prebiotic-based investigational supplements (having the compositions shown in Tables 1 to 3 below) or a control supplement (having the composition shown in Table 4 below) over a 56-day period.

**Table 1 - Powdered formulation (16.5 g)**

| **Nutrient** | **Amount of raw material** | **Effective Dose** |
|---|---|---|
| PDX (g) | 2.739 | 2.520 |
| GOS (g) | 3.729 | 2.568 |
| MFGM-10 (g) | - | - |
| Lactoferrin (g) | - | - |
| Maltodextrin (g) | 5.636 | - |
| Passionfruit Flavor (g) | 1.737 | - |
| Lavender Flavor (g) | 1.365 | - |
| Exberry Pink (g) | 0.993 | - |
| Silica (g) | 0.300 | - |
| **Total (g)** | **16.499** | - |

**Table 2 - Powdered formulation (16.5 g)**

| **Nutrient** | **Amount of raw material** | **Effective Dose5** |
|---|---|---|
| PDX (g) | 1.370 | 1.260 |
| GOS (g) | 1.865 | 1.284 |
| MFGM-10 (g) | 5.000 | - |
| [Sphingomyelin (mg)] | | 85.487 |
| [Total phospholipids (mg)] | | 325.463 |
| Lactoferrin (g) | 0.350 | 0.305 |
| Maltodextrin (g) | 3.521 | - |
| Passionfruit Flavor (g) | 1.737 | - |
| Lavender Flavor (g) | 1.365 | - |
| Exberry Pink (g) | 0.993 | - |
| Silica (g) | 0.300 | - |
| **Total (g)** | **16.501** | - |

**Table 3 - Powdered formulation (16.5 g)**

| **Nutrient** | **Amount of raw material** | **Effective Dose** |
|---|---|---|
| PDX (g) | 2.739 | 2.520 |
| GOS (g) | 3.729 | 2.568 |
| MFGM-10 (g) | 5.000 | - |
| [Sphingomyelin (mg)] | | 85.487 |
| [Total phospholipids (mg)] | | 325.463 |
| Lactoferrin (g) | 0.350 | 0.305 |
| Maltodextrin (g) | 0.287 | - |
| Passionfruit Flavor (g) | 1.737 | - |
| Lavender Flavor (g) | 1.365 | - |
| Exberry Pink (g) | 0.993 | - |
| Silica (g) | 0.300 | - |
| **Total (g)** | **16.500** | - |

**Table 4 - Powdered formulation (16.5 g)**

| **Nutrient** | **Amount of raw material** |
|---|---|
| Maltodextrin (g) | 12.104 |
| Passionfruit Flavor (g) | 1.737 |
| Lavender Flavor (g) | 1.365 |
| Exberry Pink (g) | 0.993 |
| Silica (g) | 0.300 |
| **Total (g)** | **16.499** |

Lactoferrin is a bovine lactoferrin. Passionfruit Flavor and Lavender Flavor are flavoring agents. Exberry Pink is a coloring agent. Silica is a flow agent. "Effective dose" means the amount of active ingredient in the raw material.

The study plan is shown in Table 5 below. PD means protocol day and SD means supplement day.

**Table 5 - Study Plan**

| **PD** | **SD** | **Event** | **Actions** |
|---|---|---|---|
| N/A | N/A | Remote pre-screen 1 of 2 - online | After responding to a recruitment advert, participants are remotely pre-screened for subjective poor sleep and, if they subjectively report poor sleep, they are emailed a link to consent and complete the PSQI and the SDS-CL-25. |
| | | | |
| | | | If PSQI score is greater than 5, then the participant progresses to the second remote pre-screen. |
| N/A | N/A | Remote pre-screen 2 of 2 - phone call | A second remote pre-screen is carried out. |
| | | | |
| | | | If they meet the inclusion criteria and do not meet the exclusion criteria, then participant progresses to the lab-based training and screening session. |
| -7 to -28 | N/A | Lab-based training visit | Blood Pressure/Heart Rate (BP/HR), Body Mass Index (BMI), Waist-to-Hip Ratio (WHR) will be assessed and demographic questions answered. A fasted baseline blood sample will also need to be provided prior to PD1. |
| | | | |
| | | | Participants will receive training on the Computerised Monitoring Assessment System (COMPASS) cognitive tests to be performed during the baseline and end-of-study lab visits, how to complete a sleep diary and how to use an actigraphy watch. |
| -7 | N/A | Email communication | Participant is instructed to wear the actigraphy watch and also complete the sleep diary for the next 7 days. |
| 1-4 | | Baseline lab visits | Participants will provide a baseline stool sample. |
| 1 | N/A | Baseline lab visit day 1 | Participants will complete baseline assessments for BP/HR, the Remote Monitoring Assessments (RMAs), the Participant Reported Outcome measures (PROs) and the COMPASS cognitive test battery. Participants will also complete a baseline assessment of diet. Participants will consume a standardised evening meal and spend their first night in the sleep lab. Polysomnography will be utilised. However, any polysomnography data for PD1 will not be interpreted as it represents acclimatisation to the novel surroundings. |
| 2 | N/A | Baseline lab visit day 2 | Participants will be free to leave the sleep lab during the day after providing a fasted blood sample, consuming a standardised breakfast in the lab and providing a BP/HR measurement. They will also be provided with a standardised lunch to consume off-site, during the day, and an actigraphy watch will be attached to the participant's non-dominant wrist to measure any sleep or exercise undertaken outside of the lab. |
| | | | |
| | | | Participants will return to the lab in the evening for their standardised evening meal, return the actigraphy watch, complete the baseline Observed Multitasking Stressor (OMS) task and overnight polysomnography measurements will be recorded. This (and all subsequent polysomnography data) will be recorded for use in the trial analyses. |
| 3 | N/A | Baseline lab visit day 3 | Participants will be free to leave the sleep lab during the day after providing a fasted blood sample, consuming a standardised breakfast in the lab and providing a BP/HR measurement. They will also be provided with a standardised lunch to consume off-site, during the day, and an actigraphy watch will be attached to the participant's non-dominant wrist to measure any sleep or exercise undertaken outside of the lab. |
| | | | |
| | | | Participants will return to the lab in the evening for their standardised evening meal, return the actigraphy watch and overnight polysomnography measurements will be recorded. |
| 4 | 1 | Baseline lab visit day 4 | Participants will consume a standardised breakfast and complete a BP/HR measurement in the lab. Participants will additionally be randomised and consume the first AM daily dose of their randomly allocated supplement. |
| | | | |
| | | | Participants are free to leave the sleep lab and will consume the PM daily dose in the evening at home. |
| 5-58 | 2-55 | Daily dose consumption | From protocol day 5 onwards, participants will consume their AM and PM doses at home, at separate times of the day. |
| 10 | 7 | Phone call | Remote monitoring assessment (RMA) |
| 17 | 14 | Phone call | RMA |
| 24 | 21 | Phone call | RMA |
| 31 | 28 | Phone call | RMA |
| 31-38 (± 3 days) | 28-35 (± 3 days) | Interim assessment | Participants will provide a stool sample during this period. |
| | | | Participants will also wear the actigraphy watch throughout this seven-day period. |
| | | | |
| 38 | 35 | Phone call | RMA |
| 45 | 42 | Phone call | RMA |
| 52 | 49 | Phone call | RMA |
| 52-58 | 49-55 | | During this seven-day period, participants will complete a sleep diary. Participants will also wear the actigraphy watch throughout this seven-day period. |
| 59-61 | 56, NA | End-of-study lab visit | Participants will provide a stool sample during this period. |
| 59 | 56 | End-of-study lab visit day 1 | Participants will return to the lab in the late afternoon and complete the end-of-study measurements: diet, the RMA measurements, the PRO measurements. BP, HR, BMI and WHR will also be measured. Participants will complete the COMPASS cognitive test battery before being provided with a standardised evening meal. After this, participants will complete the OMS task. |
| | | | |
| | | | At a time which is normal for them, participants will consume the PM dose of their allocated supplement (the final dosing of the study). Overnight polysomnography measurements will be recorded. |
| 60 | N/A | End-of-study lab visit day 2 | Participants will provide a blood sample before being provided with a standardised breakfast and giving a BP/HR reading. Participants will then be free to leave the sleep lab but standardised packed lunches will be provided and an actigraphy watch will be fitted as per PD2 and PD3. |
| | | | |
| | | | Participants will return to the lab in the late afternoon/evening for a standardised evening meal and overnight polysomnography measurements will be recorded. |
| 61 | N/A | Completion | Participants will be provided with a standardised breakfast and are then free to leave the sleep lab. |

### Study Treatment

The total daily supplement to be consumed by participants will be 33.0 g. This will be administered as 2 separate doses during the day: a 16.5 g dose to be consumed in the morning (AM dose) and a 16.5 g dose to be consumed in the evening (PM dose). Each 16.5 g dose will be provided in powder form, to be mixed with 200 mL of room temperature water in a shaker bottle, shaken for 1 minute and left to stand for 2 minutes before consuming.

Participants will consume the AM dose of the 56-day treatment regimen within the laboratory on protocol day 4 (supplement day 1). They will consume the PM dose later on protocol day 4, at home. From protocol day 5 onwards, participants will consume their AM and PM doses at home, at separate times of the day. On protocol day 59 (supplement day 56) participants will consume their final AM dose at home, in the morning before coming into the sleep laboratory. They will consume the final PM dose when they arrive at the sleep laboratory that afternoon/evening.

The supplement which each participant receives will be dictated by random allocation and allocation will occur during the baseline laboratory visit, on protocol day 4, once eligibility for randomisation is confirmed.

### Primary and secondary outcome measurements

The primary outcome will be the change in sleep quality after 56 days of daily consumption of one of the three test prebiotic supplements, in particular the change in sleep efficiency. This will be measured using laboratory-recorded polysomnography and sleep-related participant-recorded outcomes (PROs) during laboratory visits, actigraphy via actiwatch during laboratory visits and in the interim week and weekly remote monitoring assessments (RMAs) by telephone, as outlined in Table 5 above.

The secondary outcomes will be changes in cognitive performance (executive function, working memory, spatial memory, episodic memory, attention, mental processing speed), stress response (state anxiety, galvanic skin response (GSR), heart rate (HR), cortisol, alpha-amylase, cognitive task performance and mood), the gut microbiome (16s sequencing of gut bacterial profile from stool samples collected during baseline and end-of-study laboratory visits as well as the interim sample) and immune function (identification of blood biomarkers of immune function in samples collected at baseline and end-of-study laboratory visits) after 56 days of daily consumption of one of the three test prebiotic supplements.

All outcomes, where applicable, will be subject to baseline comparison between treatment groups. All of the outcome measures will be included in the end of trial analyses.

### Methods

### 1) Sleep

### a) Polysomnography

Participants will be monitored using polysomnography during the overnight lab-based sleep assessments. The following parameters will be measured:
- Total sleep time;
- Sleep efficiency;
- Sleep onset latency;
- Number of awakenings (number of <15 second bouts of wake surrounded, at both ends, by any sleep stage);
- Wake after sleep onset (cumulative minutes of scored wake during entire sleep duration following sleep initiation);
- Time spent in stages of REM sleep (percentage of time spent in REM sleep) and NREM sleep (percentage of time spent in N1, N2 and N3 sleep);
- REM Rebound (length, frequency and depth of REM sleep); and
- Sleep microstructure (spindle counts, k-complex counts, dichotomy data for slow versus fast spindles)

All data (with the exception of N3, which will be scored as Stage 3 and Stage 4 according to Rechtschaffen & Kales (R&K) guidelines) will be scored using American Academy of Sleep Medicine (AASM) criteria. Mean averages for the polysomnography data will be derived at each timepoint (excluding data measured overnight on protocol day 1). Primary analyses (descriptive and comparisons, using analyses of variance (ANOVAs), by group x time) will be undertaken. Effect sizes will be calculated and benchmarked against Cohen's criteria to examine both time vs group intervention outcomes.

### b) Actigraphy

A GENEactiv device will be worn on the non-dominant wrist. This device uses a triaxial capture and provides both raw, unfiltered data, or outcome data scored according to Webster's algorithm. The data collection epoch will be set to 30 seconds at a rate of 5 Hz, following bio-calibration. The following parameters will be measured:
- Sleep onset latency (recorded minutes taken from intention to sleep, from sleep diary, to Webster's derived sleep initiation);
- Total sleep time (recorded minutes asleep over entire sleep period);
- Number of awakenings (recorded number of Webster-defined awakenings during entire sleep period);
- Wake after sleep onset (recorded minutes awake during the entire sleep period following sleep onset); and
- Sleep efficiency (percentage of recorded time asleep, Webster derived, over recorded time in bed).

All data will be scored using manufacturer's guidelines with Webster's algorithm applied (using 30 second epochs). Mean averages for all data from the actigraphy (minutes for sleep latency, total sleep time and wake after sleep onset, percentages for sleep efficiency, number for number of awakenings and timepoints for M10 and L5) will be derived at each timepoint. Secondary / exploratory analyses (descriptive and comparisons, using ANOVAs, by group x time) will be undertaken subject to parametric assumptions being met.

### c) Participant Reported Outcome measures (PROs)

The following PRO measures will be recorded during the baseline and end-of-study laboratory visits:
- Pittsburgh Sleep Quality Index (PSQI) - A 19-item measure of sleep quality. Scoring creates 7 component scores: subjective sleep quality, sleep latency, sleep duration, sleep efficiency, sleep disturbance, daytime dysfunction and use of sleep medication (each on a scale of 0-3) which can be summed to create a Global PSQI score (range 0-21) with scores 5 and above indicating a 'clinically relevant sleep disturbance';
- Perceived Stress Scale (PSS) - A 14-item measure of perceived stress over the previous month (Cohen et al., Journal of Health and Social Behavior, Vol. 24, No. 4 (Dec., 1983), 385-396). Each item is rated (each on a scale from 0-4) which are summed to create a single value (range 0-40) with higher scores indicating higher levels of perceived stress;
- Profile Of Mood States (POMS) - A 65-item measure of current mood. Each item is rated (each on a scale from 0-4) which are split into positive and negative mood items and are summed (Heuchert et al., POMS 2 Manual: Profile of Mood States. Toronto, ON: Multi-Health Systems Inc. (2012) doi: 10.1037/t05057-000). To determine current negative mood both negative and positive items are summed and then positive scores are subtracted from the negative score;
- Depression, Anxiety and Stress Scale - 21 Items (DASS-21) - A 21-item measure of mood over the previous week (Lovibond, S.H. & Lovibond, P.F. (1995). Manual for the Depression Anxiety Stress Scales. (2nd. Ed.) Sydney: Psychology Foundation). Each item is rated on scale from 0-3. Scoring creates 3 component scores: depression, anxiety and stress (each on a scale of 0-21) with higher scores indicating higher symptomology. Cut-off scores, according to each set of symptoms, are available (normal, mild, moderate, severe and extremely severe) or total scores can be derived by multiplying the sum of all three component scores by 2;
- State-Trait Anxiety Inventory (STAI) - A 40-item measure of current and general anxiety levels (Spielberger, C. D. (1983). Manual for the State-Trait Anxiety Inventory (STAI). Palo Alto: Consulting Psychologists Press). Each item is rated on a scale from 1-4. Scoring creates two components: state anxiety (20 items) and trait anxiety (20 items), with a range for each between 20-80. Following transformation through reversed coding, higher scores indicate higher levels of anxiety;
- Work Productivity and Activity Impairment questionnaire (WPAI) - A 6-item measure of the impact of an individual's health on their work over the previous week. There are 4 component scores: presenteeism, activity impairment, absenteeism and overall work productivity. All items have varying response formats and higher scores indicate more impairment in each domain. Overall scores can also be summed and multiplied by 100 for an overall impairment index; and
- 12-Item Short Form Survey (SF-12) - A 12-item measure of current perceptions of health status (Ware et al.: SF-12: How to score the SF-12 physical and mental health summary scales. Health Institute, New England Medical Center.1995). All items have varying response formats and result in 8 component scores: 1) Limitations in physical activities because of health problems, 2) Limitations in social activities because of physical or emotional problems, 3) Limitations in usual role activities because of physical health problems, 4) Bodily pain, 5) General mental health (psychological distress and well-being), 6) Limitations in usual role activities because of emotional problems, 7) Vitality (energy and fatigue) and 8) General health perceptions. Scoring is automated online by publisher and expressed against normative values; and
- Gastrointestinal symptoms questionnaire - This questionnaire is to be completed at the start and end of the trial, during the baseline and end-of-study lab visits respectively. When completing the assessment, participants will answer in relation to their experiences in the previous 7 days. Total scores can range between 0-44 with a higher score indicating a more negative experience of symptoms.

The sleep diary will be recorded for one week prior to any lab visits and also during the inlab visit periods. The sleep diary is a 12-item measure of perceived sleep continuity, which is to be completed 30 minutes post awakening each morning.

In the sleep diary, sleep quality is recorded - 4 items (each scored on a 0-4 scale) covering nocturnal physical and psychological tension, sleep enjoyment and feelings of restedness. Items can be summed (range 0-16) with higher scores indicating poorer sleep quality.

The following variables are captured using the actiwatch and also recorded in the diary:
- Sleep Latency (how long, in minutes, the individual felt it took them to fall asleep after intending to sleep);
- Time in Bed (how long, in minutes, the individual reports being in bed intending to sleep);
- Number of Awakenings (number of perceived awakenings over the sleep period);
- Wake After Sleep Onset (how long, in minutes, the individual reports being awake during the night after sleep initiation);
- Total Sleep Time (how long, in minutes, the individual reports being asleep during the night between initiation and termination of sleep, accounting for nocturnal wake periods); and
- Sleep Efficiency (Total Sleep Time divided by Time in Bed x 100, expressed as a percentage).

Sleep diary data will be scored in accordance with the guidelines from the Consensus Sleep Diary (Carney et al., Sleep. 2012; 35(2): 287-302) and scores will be averaged for each week the diary is completed.

All psychometric scales will be scored according to the original scale instructions and psychometric properties within the current population will be derived from baseline data. Secondary / exploratory analyses (descriptive and comparisons, using ANOVAs, by group x time) will be undertaken subject to parametric assumptions being met. Sleep diary data will be scored in accordance with the guidelines from the Consensus Sleep Diary and will be averaged over the course of each week it is administered. Secondary / exploratory analyses (descriptive and comparisons, using ANOVAs, by group x time) will be undertaken subject to parametric assumptions being met. Where possible, sleep discrepancy values using objective (via polysomnography and/or actigraphy) and subjective (sleep diary) sleep data will be created and compared.

### d) Remote Monitoring Assessments (RMAs)

The following RMA measures will be collected via the weekly phone calls:
- Perceived sleep quality, stress, mood and productivity over the past week via numerical rating scales. This will generate individual scores of 0-4:
   ▪ Over the past week, how would you rate the overall quality of your sleep (0= 'Extremely poor' to 4= 'Extremely good')
   ▪ Over the past week, how would you rate your overall mood (0= 'Extremely poor' to 4= 'Extremely good')
   ▪ Over the past week, how would you rate your overall stress levels (0= Extremely stressed to 4= 'Not stressed at all')
   ▪ Over the past week, how would you rate your overall productivity (0= 'Extremely unproductive' to 4= 'Extremely productive')

All data will be scored using AASM criteria. Mean averages for the data (minutes for sleep latency, total sleep time, wake after sleep onset, REM latency - percentages for sleep efficiency, time spent in each sleep stage - numbers for number of awakenings, spindle counts and k-complex counts) will be derived at each timepoint (excluding adaptation lab night data). Secondary / exploratory analyses (descriptive and comparisons, using ANOVAs, by group x time) will be undertaken subject to parametric assumptions being met. Comparisons to averaged baseline and end point polysomnography data will also be undertaken. All other data from this section will be used to determine possible moderators, mediators and confounders of treatment outcomes.

### 2) Stress - Observed Multitasking Stressor (OMS)

The OMS is a stressor paradigm developed by Northumbria University, UK that involves the completion of demanding cognitive tasks which are projected on to the wall behind the participant and observed by a panel of researchers.

The OMS comprises verbal completion of three serial subtraction tasks (3, 7, and 17's) for 4 minutes each (12 minutes in total). Participants are instructed to count backwards from a given randomly generated number between 800 and 999 aloud, as quickly as possible. Performance of the task is scored for the total number of correct subtractions and incorrect subtractions. In the case of incorrect responses, subsequent responses are scored as correct if they are correct in relation to the new number. During the serial subtraction tasks, participants also complete a computerised tracking task, in which they use the mouse to move a cursor to attempt to track an asterisk that followed a smooth, random on-screen path; participants are instructed to keep the cursor as close to the asterisk as possible.

Participants will complete the STAI-state and Bond-Lader (Bond, A.; Lader, M. Use of analog scales in rating subjective feelings. Br. J. Med Psychol. 1974, 47, 211-218) mood scales before and after the completion of three four-minute verbal serial subtraction tasks.

The following physiological parameters will be recorded to measure objective stress:
- Galvanic skin response (GSR): baseline measurements will be taken shortly before the observed multitasking stressor (OMS) and the GSR will be recorded throughout the OMS;
- Heart rate (HR): baseline measurements will be taken shortly before the OMS and HR will also be recorded throughout the OMS; and
- Salivary cortisol and alpha amylase: Saliva samples will be taken from participants before and after each completion of the observed multitasking stressor (OMS).

Two-way ANOVAs will compare cognitive task performance outcomes during the OMS across treatment (x4; placebo and three treatment doses) and time (x2; baseline and end-of-study).

Two-way ANOVAs will compare STAI (State only), cortisol and alpha amylase outcomes during the OMS across treatment (x4; placebo and three treatment doses) and time (x4; baseline pre- and post-OMS and end-of-study pre- and post-OMS).

GSR and HR will first be converted to change from baseline measurements taken before each OMS. A two-way ANOVA will then compare HR and GSR across treatment (x4; placebo and 3 treatment doses) and 'epoch' during the OMS.

### 3) Cognition - COMPASS Cognitive Test Battery

### a) Visual / Associative Memory Tasks

Name to Face Recall: Twelve faces with first and last names are presented one at a time during the stimulus presentation period. During the delayed recall/recognition period participants are presented with the faces and choose from four first and four last names. Outcomes measured: accuracy and speed of response.

Picture Recognition: Fifteen pictures are presented one at a time during the stimulus presentation period. During the delayed recall/recognition period the same 15 pictures, plus 15 distractor pictures are presented, with participants making a yes/no response indicating whether the picture was in the original set. Outcomes measured: speed and accuracy of performance.

### b) Spatial and Working Memory

'Sternberg' Numeric Working Memory task: Five single digits are presented sequentially for the participant to hold in memory. Thirty probe digits (15 targets and 15 distractors) appear on screen and the participant makes a yes/no response. The task is repeated three times. Outcomes measured: speed and accuracy of performance.

'Corsi Blocks': Nine blue squares on a black background are displayed on the screen. Some of the blue squares change to red and back to blue again in a sequence. Participants are required to remember this sequence. The task is repeated five times at each level of difficulty with the sequence span increasing from 4 upwards, until the participant can no longer correctly recall the sequences.

### c) Executive Function and Verbal Memory Tasks

Peg and Ball: Participants move coloured balls on pegs using the mouse and cursor to match a target display. Difficulty increases from problems requiring 3 moves, to 4 moves and 5 moves. Outcomes measured: accuracy, thinking time and speed of performance.

Delayed Word Recognition: Participants respond yes/no to 15 words that had been presented during the stimulus presentation period, plus 15 novel distractor words. Outcomes measured: accuracy and speed of response.

Immediate and Delayed Word Recall: Participants write down as many of the 15 words that they were presented during the stimulus presentation period immediately after this period and also during the delayed recall/recognition period. Outcome measured: accuracy (% correct during each phase).

### d) Attention

Choice Reaction Time: Participants respond as quickly as possible to a stimulus (upwards pointing arrow). Outcome: speed of response.

### e) Cognitive Demand Battery

The objective of this battery is to assess the impact of interventions on speed/accuracy and mental fatigue during continuous performance of cognitively demanding tasks. The battery is detailed below.

Participants complete the 10 minute battery of tasks 3 times in immediate succession (i.e. for a continuous period of 30 minutes). Application of this battery has been shown to reliably increase self-ratings of 'mental fatigue' and to be sensitive to a number of herbal and natural interventions (Kennedy *et al.,* 2008; Kennedy & Scholey, 2004; Reay, Kennedy, & Scholey, 2005, 2006a, 2006b). The 10 minute battery comprises the following tasks.

Serial Threes Subtraction task (2 mins): Computerised versions of the serial subtraction tasks will be implemented using tests of 2-minute duration. Participants are required to count backwards in threes from a given number as quickly and as accurately as possible using the number keys to enter each response. A random starting number between 800 and 999 is presented on the computer screen, which is cleared by the entry of the first response. The task is scored for number of correct responses and number of errors. In the case of incorrect responses subsequent responses are scored as positive if they were scored as correct in relation to the new number.

Serial Sevens Subtraction task (2 mins): This is identical to the serial threes task with the exception that it involves the serial subtraction of sevens.

Rapid Visual Information Processing task (RVIP-5 mins): The participant is required to monitor a continuous series of digits for targets of three consecutive odd or three consecutive even digits. The digits are presented at the rate of 100 per minute and the participant responds to the detection of a target string by pressing the response button as quickly as possible. The task is continuous and lasts for 5 minutes, with 8 correct target strings being presented in each minute. The task is scored for percentage of target strings correctly detected, average reaction time for correct detections, and number of false alarms.

'Mental fatigue' visual analogue scale: Participants rate their current subjective 'mental fatigue' state by making a mark on a 100 mm line with the end points labelled 'not at all' (left hand end) and 'very much so' (right hand end).

### f) Statistical Analysis of Cognitive Tasks

Two-way ANOVAs will compare cognitive task outcomes across treatment (x4: placebo and three treatment arms) and time (x2: baseline and end-of-study laboratory visits). Additionally, outcomes derived from individual tasks can be collapsed into five global cognitive domains (accuracy of attention, speed of attention, working memory, speed of memory & episodic memory) and an additional two domains of 'overall speed' and 'overall accuracy'; which will also be analysed according to the above plan.

### Example 2 - Preclinical Study

The aim of this study was to investigate the extent to which early life dietary oligosaccharides and/or polydextrose (PDX+GOS) modulate sleep and protect sleep architecture following stress.

Timed-pregnant female Sprague Dawley rats were purchased from Charles River Laboratories International, Inc., (Hollister, CA and Canada). Male pups were weaned at postnatal day 21 (P21) and randomised into one of five treatment groups (see Table 6 below). All rats were housed in a temperature-controlled recording room under a 12 hour light: dark cycle with food and water available ad libitum. Environmental conditions were regulated, and room temperature (24±2°C), humidity (50±20% relative humidity), and lighting were monitored and recorded. Animals were inspected daily.

All experimenters were blind to type of diet administered to each group. The diets were formulated by Mead Johnson Nutrition (MJN, Evansville IN) based on AIN-93G specifications to be isocaloric and have similar carbohydrate, protein, fat, vitamin and mineral levels. Experimental diets contained 7.0 g/kg galactooligosaccharides (GOS), 7.0 g/kg polydextrose (PDX) and/or 2.2 g/kg sialyllactose (SL). Diets were produced by Envigo (Indianapolis, IN), and ingredients were supplied by: PDX (Danisco, Terre Haute, IN), GOS (Friesland Campina, Zwolle, The Netherlands) and SL (SAL-10; Aria Foods Ingredients, Aarhus, Denmark).

A total of n=105 male Sprague Dawley rats were used in this study with a subset of animals (n=40) used for sleep/wake analysis. Upon weaning (P21), rats were randomised into one of five treatment groups, based upon diet and exposure to a stressor, as follows:

**Table 6**

| **Group number** | **PDX+GOS** | **SAL** | **Stressor** |
|---|---|---|---|
| 1 | - | - | - |
| 2 | - | - | + |
| 3 | + | + | + |
| 4 | + | - | + |
| 5 | - | + | + |

The experimental protocol consisted of two phases, a diet manipulation phase and a sleep-stressor phase. During the diet manipulation phase, rat weanlings were randomised to either the control diet or one of the three formulated diets. Rats were provided these diets from age P21 to P51, after which all animals were given standard chow. During this diet-manipulation phase, body weights were recorded twice per week and food consumption determined for weekly intervals.

The second phase of the protocol consisted of telemetric recordings, exposure to stressor, and behavioural assessment. Telemeters were implanted on P64 and undisturbed baseline recordings obtained on P84.

Rats in groups 2 to 5 were exposed to inescapable shock stress (uncontrollable stress paradigm) on P85 using previously published protocols (Greenwood et al., Behav Brain Res. 2012 Aug 1; 233(2): 314-321). Briefly, rats were restrained in Plexiglass tubes and received ~100, 5 s tail shocks (1.5 mA) on a 1-min variable-interval schedule. Stress protocols were conducted during the beginning of the light period. After the end of the stress protocol, rats were returned to their home cages. Home-cage control animals (no stress) remained undisturbed during this period. The results are shown in Figure 1 and in Table 7.

**Table 7. Sleep parameters determined from rats during baseline and post-stressor**

| **Sleep Parameter and Condition** | **Light period (ZT5-12)** | | **Dark period (ZT13-24)** | |
|---|---|---|---|---|
| | **Baseline** | **Post-stress** | **Baseline** | **Post-stress** |
| **NREMS duration (% recording time)** | | | | |
| G1 Control Diet | 52.1 ± 3.4 | 51.6 ± 3.4 | 29.9 ± 1.7 | 32.0 ± 1.9 |
| G2 Control Diet+Stress | 51.1 ± 3.1 | 54.9 ± 3.9 | 30.2 ± 1.9 | [40.2 ± 1.6*] |
| G3 PDX+GOS+SL+Stress | 50.5 ± 2.3 | 59.2 ± 2.4 | 28.6 ± 2.7 | [41.4 ± 2.3*] |
| G4 PDX+GOS+Stress | 54.1 ± 1.8 | 58.8 ± 1.7 | 28.5 ± 3.4 | [46.4 ± 2.4**+] |
| G5 SL+Stress | 51.0 ± 3.1 | 56.1 ± 2.1 | 30.1 ± 3.0 | [43.7 ± 1.7**] |

| **NREMS bouts (number/h)** | | | | |
|---|---|---|---|---|
| G1 Control Diet | 19.7 ± 1.5 | 20.5 ± 1.5 | 12.1 ± 0.6 | 13.3 ± 1.0 |
| G2 Control Diet+Stress | 21.1 ± 2.0 | 16.7 ± 1.4 | 12.8 ± 1.1 | 15.6 ± 0.6 |
| G3 PDX+GOS+SL+Stress | 19.7 ± 1.4 | 17.1 ± 1.0 | 10.9 ± 1.0 | 15.9 ± 0.6 |
| G4 PDX+GOS+Stress | 19.7 ± 1.7 | 15.6 ± 1.2 | 11.4 ± 0.8 | 16.1 ± 0.6** |
| G5 SL+Stress | 20.1 ± 1.5 | 16.8 ± 1.1 | 11.9 ± 1.1 | 17.7 ± 1.3 |

| **NREMS bout duration (min)** | | | | |
|---|---|---|---|---|
| G1 Control Diet | 1.5 ± 0.1 | 1.5 ± 0.1 | 1.3 ± 0.1 | 1.3 ± 0.1 |
| G2 Control Diet+Stress | 1.5 ± 0.1 | 2.1 ± 0.1** | 1.4 ± 0.1 | 1.5 ± 0.1 |
| G3 PDX+GOS+SL+Stress | 1.5 ± 0.1 | 2.1 ± 0.1** | 1.4 ± 0.1 | 1.5 ± 0.1 |
| G4 PDX+GOS+Stress | 1.7 ± 0.1 | 2.3 ± 0.1** | 1.4 ± 0.1 | 1.8 ± 0.1** |
| G5 SL+Stress | 1.5 ± 0.1 | 2.1 ± 0.1** | 1.3 ± 0.1 | 1.5 ± 0.1 |

| **REMS duration (% recording time)** | | | | |
|---|---|---|---|---|
| G1 Control Diet | 12.9 ± 1.1 | 11.6 ± 0.8 | 5.2 ± 0.8 | 5.6 ± 1.1 |
| G2 Control Diet+Stress | 10.4 ± 0.6 | 11.9 ± 1.3 | 5.4 ± 0.8 | **[11.6 ± 0.9**]** |
| G3 PDX+GOS+SL+Stress | 11.6 ± 1.4 | 13.8 ± 1.5 | 5.2 ± 0.8 | **[11.5 ± 1.0**]** |
| G4 PDX+GOS+Stress | 11.5 ± 1.3 | 11.9 ± 1.4 | 6.4 ± 0.9 | **[14.1 ± 1.5**]** |
| G5 SL+Stress | 12.6 ± 1.3 | 14.4 ± 1.7 | 6.0 ± 0.9 | **[12.3 ± 1.0**]** |

| **REMS bouts (number/h)** | | | | |
|---|---|---|---|---|
| G1 Control Diet | 4.8 ± 0.4 | 4.5 ± 0.3 | 2.3 ± 0.4 | 2.6 ± 0.5 |
| G2 Control Diet+Stress | 3.9 ± 0.3 | 4.2 ± 0.5 | 2.3 ± 0.4 | **[4.9 ± 0.4*]** |
| G3 PDX+GOS+SL+Stress | 4.3 ± 0.5 | 5.3 ± 0.7 | 2.3 ± 0.3 | **[4.9 ± 0.4*]** |
| G4 PDX+GOS+Stress | 4.4 ± 0.3 | 4.2 ± 0.5 | 2.8 ± 0.3 | **[5.6 ± 0.6**]** |
| G5 SL+Stress | 5.3 ± 0.6 | 5.3 ± 0.7 | 2.8 ± 0.4 | **[5.5 ± 0.5**]** |

| **REMS bout duration (min)** | | | | |
|---|---|---|---|---|
| G1 Control Diet | 1.4 ± 0.1 | 1.5 ± 0.1 | 0.9 ± 0.1 | 0.9 ± 0.1 |
| G2 Control Diet+Stress | 1.5 ± 0.1 | 1.7 ± 0.1 | 1.0 ± 0.1 | **[1.3 ± 0.1*]** |
| G3 PDX+GOS+SL+Stress | 1.3 ± 0.1 | 1.5 ± 0.1 | 0.9 ± 0.1 | **[1.3 ± 0.1]** |
| G4 PDX+GOS+Stress | 1.4 ± 0.1 | 1.6 ± 0.1 | 1.0 ± 0.1 | **[1.5 ± 0.1**]** |
| G5 SL+Stress | 1.2 ± 0.1 | 1.6 ± 0.0 | 0.9 ± 0.1 | **[1.3 ± 0.1*]** |

| **Wake duration (% recording time)** | | | | |
|---|---|---|---|---|
| G1 Control Diet | 35.0 ± 4.4 | 36.8 ± 3.9 | 64.9 ± 2.3 | 62.4 ± 2.6 |
| G2 Control Diet+Stress | 38.5 ± 3.3 | 33.2 ± 4.9 | 64.4 ± 2.5 | **[48.3 ± 2.1**]** |
| G3 PDX+GOS+SL+Stress | 37.9 ± 2.8 | 27.0 ± 2.4 | 66.2 ± 3.3 | **[47.1 ± 2.3**]** |
| G4 PDX+GOS+Stress | 34.5 ± 2.9 | 29.3 ± 2.7 | 65.1 ± 4.0 | **[39.5 ± 2.3**+]** |
| G5 SL+Stress | 36.4 ± 4.2 | 29.5 ± 3.5 | 63.9 ± 3.8 | **[43.9 ± 2.4**]** |

| **Wake bouts (number/h)** | | | | |
|---|---|---|---|---|
| G1 Control Diet | 11.3 ± 1.1 | 11.5 ± 1.0 | 9.6 ± 0.6 | 9.6 ± 0.9 |
| G2 Control Diet+Stress | 11.3 ± 1.0 | 7.7 ± 0.7** | 9.0 ± 0.8 | 10.4 ± 0.6 |
| G3 PDX+GOS+SL+Stress | 11.4 ± 1.0 | 7.9 ± 0.5** | 7.6 ± 0.3 | 9.4 ± 0.6 |
| G4 PDX+GOS+Stress | 10.2 ± 0.7 | 7.6 ± 0.4** | 7.6 ± 0.6 | 9.0 ± 0.8 |
| G5 SL+Stress | 10.1 ± 0.6 | 7.8 ± 0.6** | 8.3 ± 0.6 | 10.4 ± 1.1 |

| **Wake bout duration (min)** | | | | |
|---|---|---|---|---|
| G1 Control Diet | 4.4 ± 2.8 | 3.0 ± 1.2 | 9.6 ± 1.7 | 9.8 ± 1.5 |
| G2 Control Diet+Stress | 3.4 ± 1.3 | 3.8 ± 0.9 | 7.6 ± 1.4 | 5.7 ± 1.0* |
| G3 PDX+GOS+SL+Stress | 3.3 ± 0.8 | 2.7 ± 0.6 | 10.9 ± 1.9 | 6.6 ± 0.8 |
| G4 PDX+GOS+Stress | 3.2 ± 1.1 | 2.7 ± 0.6 | 9.7 ± 1.4 | 3.8 ± 0.5** |
| G5 SL+Stress | 3.5 ± 1.1 | 3.0 ± 0.7 | 10.9 ± 2.2 | 4.1 ± 0.8** |

Values are means ± SEM. Single asterisk (*) indicates a statistical difference of *p*≤0.05 whereas double asterisk (**) indicates a statistical difference of *p*<0.01 relative to values obtained from Control Diet + No Stress group. + indicates *p*≤0.05 relative to Control Diet + Stress. Values in square brackets indicate a statistical difference of *p*<0.05 between post-stress values and corresponding baseline values.

### Discussion

The diurnal distribution of sleep-wake behaviour during undisturbed baseline conditions was normal for all animals and was characterised by more NREM and REM sleep during the light period (ZT1-12), and more wakefulness during the dark (ZT13-24; Figure 1; Table 7). There were no baseline differences in sleep-wake behaviour among rats subsequently randomised to any of the diet manipulations (Figure 1, left panels).

However, post-stressor analyses revealed a significant increase in NREM and REM sleep relative to non-stressed controls for all groups, irrespective of diet (Figure 1, right panels; Table 7). The increase in NREM during the post-stress dark period was more in animals fed PDX+GOS diet (group 4) as compared to animals subjected to stress but maintained on the control diet (group 2). These increases in post-stress NREM and REM sleep during the dark period were accompanied by reduced wake (Figure 1; Table 7). The reduced wake percentage during the post-stress dark period was less in animals fed the PDX+GOS diet as compared to animals subjected to stress but maintained on control diet (Figure 1; Table 7).

Significant changes in sleep architecture were apparent during both the post-stress light period and the post-stress dark period (Table 7). Although there were no significant changes in the total amounts of sleep during the post-stress light period, NREM bout duration increased while the number of wake bouts decreased compared to the non-stressed controls indicating a consolidated pattern of NREM following stress. During the dark period following stress, REM bout durations and the number of REM bouts increased for all stress groups compared to baseline and/or non-stressed controls. The decrease in wake time during the dark following stress was primarily due to a decrease in the number of wake bouts.

For the PDX+GOS group, significantly longer NREM bouts occurred as well as an increased number of NREM bouts during the dark compared to the non-stressed controls. In addition, the increase in NREMS duration in dark period and increase in REMS bouts in dark period were more significant compared to animals fed the control diet or diet with combined SAL and PDX+GOS. Collectively, these changes suggest that stress was followed by both increased sleep and more consolidated sleep during the subsequent dark period compared to un-stressed controls.

In summary, sleep is increased in the PDX+GOS group relative to both the stressed and unstressed control groups. Increased sleep, and in particular REM sleep, has been hypothesised to be an adaptive response to stress. Thus, in summary, the results suggest that diets rich in the prebiotics PDX and GOS could enhance this adaptive response to stress. Further, it is hypothesised that the improvements in sleep outlined above would confer an improvement in cognitive function, for example by enabling the cognitive repair processes that take place during sleep.

## Claims

1. A composition for use in reducing stress and/or enhancing mood in a subject, the composition comprising a prebiotic component comprising polydextrose (PDX) and galactooligosaccharides (GOS).

2. The composition for use according to claim 1, wherein the reduction in stress is an improvement in response to psychological stress.

3. The composition for use according to claim 1 or claim 2, wherein the reduction in stress is selected from a lower resting heart rate, a lower elevation in heart rate, a higher heart rate variability (HRV), a lower increase in blood pressure, a lower increase in salivary cortisol and/or a lower increase in serum cortisol.

4. The composition for use according to claim 1, wherein the reduction in stress is a lower level of perceived stress.

5. The composition for use according to any preceding claim, wherein the composition is administered once daily or twice daily.

6. The composition for use according to any preceding claim, wherein the subject is an adult.

7. The composition for use according to any preceding claim, wherein the prebiotic component consists of PDX and GOS.

8. The composition for use according to any preceding claim, wherein a daily dosage of PDX is in the range of about 0.05 g/day to about 10.0 g/day and a daily dosage of GOS is in the range of about 0.05 g/day to about 10.0 g/day.

9. The composition for use according to any preceding claim, wherein the composition further comprises a fat or lipid source, wherein the fat or lipid source comprises polar lipids.

10. The composition for use according to claim 9, wherein the polar lipids comprise at least one of sphingomyelin, gangliosides, phospholipids, cholesterol, 7-dehydrocholesterol, or any combination thereof.

11. The composition for use according to claim 9 or claim 10, wherein the fat or lipid source is a whey protein concentrate and/or the fat or lipid source comprises milk fat globule membrane (MFGM).

12. The composition for use according to claim 11, wherein a daily dosage of MFGM is in the range of about 0.05 g/day to about 20.0 g/day.

13. The composition for use according to any preceding claim, wherein the composition further comprises lactoferrin.

14. The composition for use according to claim 13, wherein a daily dosage of lactoferrin is in the range of about 1 mg/day to about 10.0 g/day.

15. The composition for use according to any preceding claim, wherein the composition is a nutritional composition and/or wherein the composition is a liquid, a liquid concentrate, a powder or a nutritional bar.
